# EUROPEAN PATENT APPLICATION

(11) **EP 1 798 554 A1**
(43) Date of publication of application: **20.06.2007**
(21) Application number: 05027187.3
(22) Date of filing: 13.12.2005
(51) Int. Cl.: G01N 33/49, G01N 33/00

(54) **Method and system for measurement of nitrite and nitric oxide release**

(71) Applicant: Millimed A/S, 4000 Roskilde (DK)
(72) Inventor: The designation of the inventor has not yet been filed
(74) Representative: Inspicos A/S

(57) **Abstract**

A method for combined measurement of release of nitrite (NO₂⁻) and nitric oxide (NO) from a probe containing nitric oxide, such as an intravascular medical device immersed in a first vessel, comprises conveying nitric oxide (NO) released directly from the probe to a nitric oxide analysis apparatus. Nitrite is removed from a first vessel, which may e.g. comprise a head-space chamber, and transformed to nitric oxide (NO), which is conveyed to the nitric oxide analysis apparatus. A selector valve arranged upstream of the nitric oxide analysis apparatus is operated to selectively allow one of the directly released nitric oxide (NO) and the nitrite-derived nitric oxide (NO) into the apparatus. Directly released nitric oxide may be continuously flushed away from the first vessel.

## Description

### Technical field

The present invention relates to a method and system for measurement of nitrite (NO₂⁻) and nitric oxide (NO). In particular, the invention is concerned with the use of a nitric oxide analysis apparatus for the continuous measurement of nitric oxide relased from a probe and for intermittent measurement of nitrite released from the same probe.

### Background of the invention

Nitric oxide (NO) has proven to be a useful agent in a wide range of physiological processes, such as reendothelialization, vasodilation, neurotransmission and platelet aggregation. The biological function of NO has also been found to include action as cytotoxic agent. Therefore, the need for studying NO release from biological and chemical molecules has increased, e.g. for the purpose of simulating NO release from medical devices during research and development of such devices. However, NO readily reacts with oxygen and water, forming nitrite (NO₂⁻) which acts as an interfering molecule in most available methods used for measuring NO.

### Summary of the invention

It is thus an object of preferred embodiments of the present invention to provide a method and a system capable of obtaining an NO measurement, in which the presence of nitrite can be eliminated or taken into account to obtain a more precise NO measurement than hitherto achievable. It is a further object of preferred embodiments of the invention to provide a method and a system facilitating the procedure of obtaining NO and NO₂⁻ reliable measurements.

The present invention thus provides a method for combined measurement of release of nitrite (NO₂⁻) and nitric oxide (NO) from a probe containing nitric oxide, comprising the steps of:
- placing the probe in a first vessel, in which nitrite (NO₂⁻) and nitric oxide (NO) is released from the probe;
- conveying nitric oxide (NO) released directly from the probe through a first conduit to a gas feed conduit, the gas feed conduit being arranged to convey nitric oxide into a nitric oxide analysis apparatus;
- removing nitrite (NO₂⁻) from the first vessel;
- transforming the removed nitrite (NO₂⁻) to nitric oxide (NO) to obtain nitrite-derived nitric oxide, the step of transforming occurring subsequent to the step of removing nitrite from the first vessel;
- conveying the nitrite-derived nitric oxide through a second conduit to the gas feed conduit;
- operating a selector valve arranged at an upstream end of the gas feed conduit and at respective downstream ends of the first and second conduits, so as to selectively allow one of the directly released nitric oxide (NO) and the nitrite-derived nitric oxide (NO) into the gas feed conduit.

Additionally, the invention provides a system for measurement of release of nitrite and nitric oxide from a probe containing nitric oxide, the system comprising:
- a first vessel for accommodtion of the probe;
- a nitric oxide analysis apparatus;
- a gas feed conduit, through which nitric oxide may be conveyed to said analysis apparatus;
- a first conduit, through which nitric oxide released directly from the probe may be conveyed from the first vessel to an upstream end of the gas feed conduit;
- a purge vessel arranged upstream of a second conduit, whereby nitrite may be transformed to nitric oxide In the purge vessel, the second conduit being arranged to convey nitrite-derived may be conveyed to the upstream end of the gas feed conduit;
- a selector valve arranged at the upstream end of the gas feed conduit, the selector valve being connected to respective downstream ends of the first and second conduits, and arranged to selectively allow directly released nitric oxide and nitrite-derived nitric oxide into the gas feed conduit.

Thanks to the provision of a selector valve, NO release and NO₂⁻ background can be monitored in a single analytical run. Following NO gas and liquid nitrite calibrations of the nitric oxide analysis apparatus, an output mV signal of the apparatus will be indicative of actual concentrations.

In a particularly preferred embodiment, the method and system of the present invention is suitable for studying quantitative on-line release of NO combined with quantitative measurements of nitrite (NO₂⁻), using a combined setup of a dynamic head space and purge vessel system enabling precise quantitative measurements of NO release and NO₂⁻ concentrations from the same probe.

The nitric oxide analysis apparatus may comprise an apparatus known *per se*, for example a so-called high-sensitivity detector for measuring nitric oxide based on a gas-phase chemiluminescent reaction between nitric oxide and ozone:

NO + O3 -> NO2* + O2

NO2* -> NO2 + hv

Emission from electronically excited nitrogen dioxide is in the red and near-infrared region of the spectrum, and may be detected by a termoelectrically cooled, red-sensitive photomultiplier tube.

One suitable analysis apparatus is the Nitric Oxide Analyzer NOA^{™} 280i commercially available from Sievers^{®}, Boulder, Colorado, USA.

### Detailed description of the invention

Preferred embodiments of method and system of the present invention will now be described with reference to the accompanying drawings, in which:
Figs. 1 and 2 show, in schematic illustration, a first and a second embodiment of a combined setup of a dynamic head space and purge vessel system enabling precise and quantitative measurements of NO release and NO₂⁻ concentrations from the same probe;
Fig. 3 illustrates monitoring of NO release and NO₂⁻ background in a single analytical run.

### Dynamic head space

In a presently preferred embodiment of the invention, on-line detection of NO release is carried out in a dynamic head space chamber containing a defined solution, such an aqueous solution, and a NO releasing proble (also referred to as a sample). The head space chamber is continuously flushed with a controlled flow of high grade nitrogen gas. The nitrogen gas flowing through the head space chamber ensures an oxygen free solution in the head space chamber, avoiding transformation of NO Into NO₂⁻. Additionally, the N₂ gas flushing the head space chamber strips of any NO released into the solution in the head space chamber and carries the released NO to the NO analyzer. The gas flow into the NO analyzer is controlled by a static frit restrictor mounted at the inlet of the analyzer while the N₂ flow into the head space chamber is controlled by an adjustable flow controller. The N₂ flow into the head space chamber is adjusted to a higher flow than the flow into the NO analyzer, ensuring no leakage of NO or O₂ from the outside atmosphere to the head space chamber. The excess gas volume is exhausted through a vent, such as a **T** connection, mounted downstream of the head space chamber. This split flow system can additionally be used for regulating the sensitivity of the NO analyzer. In cases where NO release in the head space vessel exceeds detection range of the NO analyzer, the signal can be decreased by increasing the N₂ flow into the head space vessel keeping the flow into the analyzer substantially constant. This maneuver decreases the fraction of the NO in the N₂ stream that enters the detector, resulting in a decreased signal at the detector.

### Nitrite detection unit

Measurement of nitrite is carried out in a purge vessel system continuously flushed with a controlled flow of N₂ gas to ensure oxygen free environment in the purge vessel. The vessel contains an acidic solution of sodium iodide allowing the following reaction to take place upon injection of a nitrite containing sample:

I⁻+ NO₂⁻ + 2 H⁺ -> NO + ½ I₂ +H₂O

Upon reduction of NO₂⁻ to NO, NO is drawn into the analyzer and measured as NO gas in the same detection system as used for dynamic head space measurement.

### Combined on line NO release and NO₂⁻ measurements

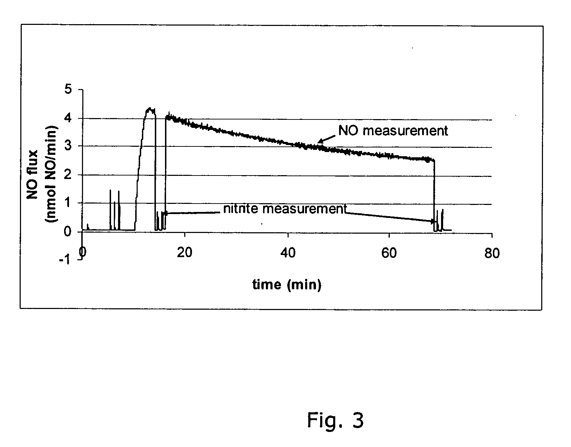

Since NO₂⁻ is an unwanted side product in most NO release systems, measuring the combined NO release and NO₂⁻ concentration in these systems is of significant importance. In the method and system of the present invention, NO release is measured in the dynamic head space chamber setup while nitrite is measured in the purge vessel setup. The two setups are combined in a selector valve, such as a 4-way stop cock, enabling the inlet to the NO analyzer to be switched between dynamic head space and purge vessel setup. By switching from dynamic head space to purge vessel setup during a NO measurement and withdrawing a sample from the head space chamber and injecting it into the purge vessel system it is possible to monitor both NO release and NO₂⁻ background in a single analytical run as shown in Fig. 3. Following NO gas and liquid nitrite calibrations, the mV signal obtained from the analyzer can be transformed into actual concentrations.

### Further features of the invention

The step of conveying nitric oxide (NO) may comprise continuous transport of directly released nitric oxide away from the first vessel. During such transport, the selector valve should preferably be in a position, in which nitric oxide is allowed into the analysis apparatus. Nitrogen (N₂) may be used as a carrier gas for transport of nitric oxide (NO). A first nitrogen feed conduit may be provided, through which nitrogen (N₂) may be conveyed into the first vessel. A pressure tank containing N₂ may be used as a N₂ source, whereby excess pressure in the pressure tank provides a pressure gradient in the system, which ensures appropriate transport of gasses. The flow rate of nitrogen (N₂) in the nitrogen feed conduit may be measured by means of a flow meter arranged in the nitrogen feed conduit, the flow meter producing an output signal, which is passed to the analysis apparatus.

The first vessel may comprise a continuously flushed headspace chamber, in which case the nitric oxide analysis apparatus may continuously analyse gas fed to the apparatus via the gas feed conduit.

The probe may be is immersed in an aqueous solution, e.g saline or blood. With regard to analysing of NO and NO₂⁻ release from medical devices, in particular implants, it may be desired that the conditions of the aqueous solution resemble the physiological conditions of the human or animal body in the best possible way. For example, the viscosity of the solution may be close to that of blood, and the solution may be maintained at body temperature, i.e. at approximately 37°C.

A pressure above atmospheric pressure may be maintained in the first conduit in order to prevent atmospheric air from entering the system. Thus, in particular ingress of oxygen may be prevented. Excess gas in flow in the first conduit may be released through a vent in the fist conduit arranged downstream of the first vessel (or head space chamber) and preferably upstream of the selector valve.

The step of conveying nitrite (NO₂⁻) may comprise sampling at least one nitrite sample from the first vessel and conveying the sample to the second conduit, following transformation into nitric oxide. Transformation may e.g. occur in the purge vessel. The nitrite sample may be conveyed into the purge vessel arranged uptream of the second conduit. Nitrogen (N₂) may be used as a carrier gas for transport of nitrite from the purge vessel through the second conduit and the gas feed conduit. In one embodiment, the nitrite sample is conveyed manually from the first vessel to the purge vessel. In another embodiment, transport of the nitrite sample is effected by a pump system or an auto-sampling system. In such an embodiment, operation of the pump or auto-sampling system is preferably synchronized with operation of the selector valve, so that the nitrite sample is only conveyed into the second conduit when the selector valve is in a position allowing the flow of nitrite into the analysis apparatus.

The probe may comprise a medical device, such as an intermittent or permanent intravascular implant, such as a stent, a stent graft, a balloon, a balloon catheter, a guidewire, or en embolization device. The medical device may incorporate or be coated with nitric oxided or with a coating material, such as a suitable polymer, loaded with NO or a NO-releasing agent.

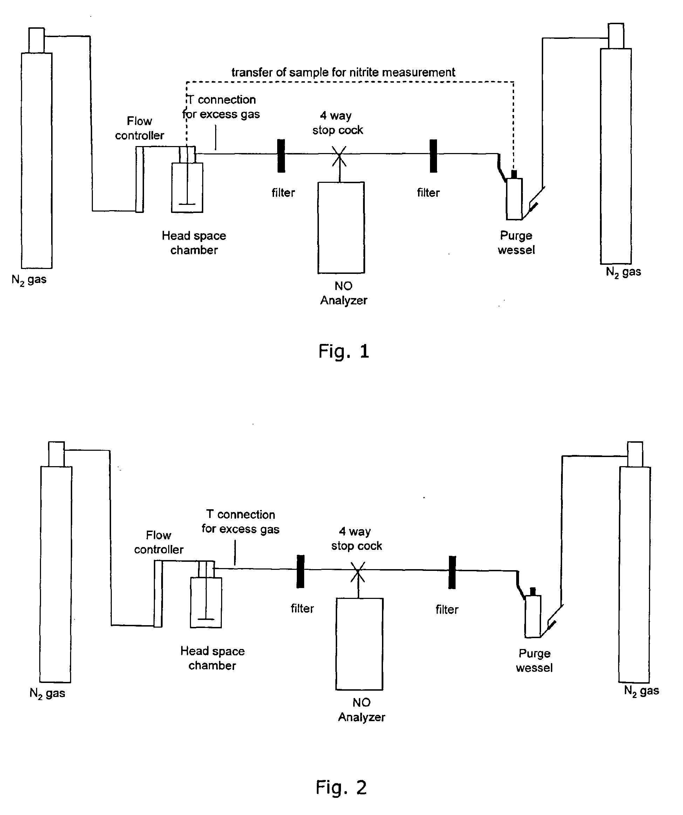

In the embodiment of Fig. 2, no conduit is provided to connect the head space chamber with the purge vessel. It is rather contemplated that, in this embodiment, nitrite will be conveyed by manual means.

## Claims

1. A method for combined measurement of release of nitrite (NO₂⁻) and nitric oxide (NO) from a probe containing nitric oxide, comprising the steps of:
- placing the probe in a first vessel, in which nitrite (NO₂⁻) and nitric oxide (NO) is released from the probe;
- conveying nitric oxide (NO) released directly from the probe through a first conduit to a gas feed conduit, the gas feed conduit being arranged to convey nitric oxide into a nitric oxide analysis apparatus;
- removing nitrite (NO₂⁻) from the first vessel;
- transforming the removed nitrite (NO₂⁻) to nitric oxide (NO) to obtain nitrite-derived nitric oxide, the step of transforming occurring subsequent to the step of removing nitrite from the first vessel;
- conveying the nitrite-derived nitric oxide through a second conduit to the gas feed conduit;
- operating a selector valve arranged at an upstream end of the gas feed conduit and at respective downstream ends of the first and second conduits, so as to selectively allow one of the directly released nitric oxide (NO) and the nitrite-derived nitric oxide (NO) into the gas feed conduit.

2. A method according to claim 1, wherein the step of conveying directly released nitric oxide (NO) comprises continuous transport of nitric oxide away from the first vessel when the selector valve is In a position, in which nitric oxide is allowed into the analysis apparatus.

3. A method according to claim 2, wherein nitrogen (N₂) is used as a carrier gas for transport of directly released nitric oxide (NO).

4. A method according to claim 3, further comprising a first nitrogen feed conduit, through which nitrogen (N₂) is conveyed into the first vessel.

5. A method according to claim 4, wherein the flow rate of nitrogen (N₂) in the nitrogen feed conduit is measured by means of a flow meter arranged in the nitrogen feed conduit, the flow meter producing an output signal, which is passed to the analysis apparatus.

6. A method according to any of the preceding claims, wherein the first vessel comprises a continuously flushed headspace chamber.

7. A method according to any of the preceding claims, wherein the probe is immersed in an aqueous solution.

8. A method according to claim 7, wherein the aqueous solution is maintained at a temperature of approximately 37°C.

9. A method according to any of the preceding claims, wherein a pressure above atmospheric pressure is maintained in the first conduit.

10. A method according to claim 9, further comprising the step of releasing a portion of the gas flow in the first conduit through a vent in the first conduit.

11. A method according to any of the preceding claims, wherein the step of conveying nitrite (NO₂⁻) comprises the step of sampling at least one nitrite sample from the first vessel and conveying the sample to a purge vessel arranged upstream of the second conduit, and wherein the step of transforming nitrite to nitric oxide is carried out in said purge vessel.

12. A method according to claim 11, wherein nitrogen (N₂) is used as a carrier gas for transport of nitrite from the purge vessel through the second conduit and the gas feed conduit.

13. A method according to claim 11 or 12, wherein the nitrite sample is conveyed manually from the first vessel.

14. A method according to claim 11 or 12, wherein transport of the nitrite sample is effected by a pump system or an auto-sampling system, and wherein operation of the pump system is synchronized with operation of the selector valve.

15. A method according to any of the preceding claims, wherein the probe comprises a medical device.

16. A method according to claim 15, wherein the medical device comprises an intermittent or permanent intravascular implant.

17. A method according to claim 15, wherein the medical device is selected from the group consisting of: a stent, a stent graft, a balloon, a balloon catheter, a guidewire, and en embolization device.

18. A system for measurement of release of nitrite and nitric oxide from a probe containing nitric oxide, the system comprising:
- a first vessel for accommodtion of the probe;
- a nitric oxide analysis apparatus;
- a gas feed conduit, through which nitric oxide may be conveyed to said analysis apparatus;
- a first conduit, through which nitric oxide released directly from the probe may be conveyed from the first vessel to an upstream end of the gas feed conduit;
- a purge vessel arranged upstream of a second conduit, whereby nitrite may be transformed to nitric oxide in the purge vessel, the second conduit being arranged to convey nitrite-derived may be conveyed to the upstream end of the gas feed conduit;
- a selector valve arranged at the upstream end of the gas feed conduit, the selector valve being connected to respective downstream ends of the first and second conduits, and arranged to selectively allow directly released nitric oxide and nitrite-derived nitric oxide into the gas feed conduit.
